# EUROPEAN PATENT APPLICATION

(11) **EP 4 834 276 A1**
(43) Date of publication of application: **04.11.2026**
(21) Application number: 26176019.3
(22) Date of filing: 30.04.2026
(51) Int. Cl.: A61C 19/045, A61B 34/10, A61C 7/00, A61C 13/00, G06N 3/02, G16H 20/40, G16H 30/40, G16H 50/20, G16H 50/50

(54) **METHOD FOR PLANNING A DENTAL TREATMENT, INSTALLATION AND COMPUTER PROGRAM**

(30) Priority: 02.05.2025 EP 25174117
(71) Applicant: Roviba Dental Sarl, 1201 Geneve (CH)
(72) Inventor: LAGRANGE, Baptiste, 26000 Valence (FR); DOLIVEUX, Romain, 79102 Freibourg-im-Breisgau (DE); SIXDENIER, Vivien, 69250 POLEYMIEUX-AU-MONT-D'OR (FR)
(74) Representative: Fidal Innovation

(57) **Abstract**

Method (10) for planning a dental treatment on an individual, the method (10) comprising digitally and automatically obtaining (12) a wax-up built based on a determined performance of the jaw function of the individual, the wax-up comprising a proposed position and/or shape of the individual's intraoral structures.

## Description

The present disclosure relates to a method for planning a dental treatment on an individual, and to an installation for carrying out this method. The present disclosure further relates to a computer program that cause a computer to carry out the disclosed method for planning a dental treatment.

### BACKGROUND

In the realm of modern dentistry, achieving a perfect smile is both an art and a science. Dental treatments have evolved significantly, offering a variety of solutions to enhance the aesthetics of teeth. One such advanced technique is the wax-up.

A wax-up involves creating a detailed, three-dimensional model of a patient's teeth that allows dentists to visualize and plan the treatment with precision. Classically, wax-ups are created using wax. Today, digital wax-ups are also available. However, the methods and devices used to obtain a wax-up or digital wax-up focus on achieving a perfect aesthetic position of the teeth, namely the six upper front teeth (also known as the "social six"), forgetting that intraoral structures are not only an aesthetic feature of each individual, but a main element of the manducatory apparatus that must function correctly to enhance quality of life. Focusing dental treatments solely on aesthetic results leads to failure of the treatment, which can manifest itself in the form of broken prostheses, the need for further orthodontic treatment, failure of the osseointegration of implants and/or root fractures of the teeth, among others.

Furthermore, known methods and systems for obtaining digital wax-up require physical, mechanical or electronic devices as well as human intervention to track jaw movements, thus complicating the system or method.

The present disclosure aims to propose a solution that reduces the risk of encountering such problems.

### SUMMARY

Thus, the disclosure relates to a method for planning a dental treatment on an individual, the method comprising digitally and automatically obtaining a wax-up built based on a determined performance of the jaw function of the individual, the wax-up comprising a proposed position and/or shape of the individual's intraoral structures.

In particular, the wax-up may be obtained either by generating a new digital wax-up from the determined performance of the jaw function, or by modifying an existing wax-up previously created for the individual.

The obtained wax-up takes therefore into account the performance of the jaw function for proposing the position and/or shape of the individual's intraoral structures. In other words, the proposed method proposes a workflow which results in a wax-up based on the physiology of the individual's jaw, this wax-up being then used to plan the dental treatment. The wax-up is therefore not only based on aesthetic aspects of the smile, but also on the other parameters that have an impact on the satisfactory functioning of the individual's jaw and so, of the manducatory apparatus. The chances of success and durability of the planed dental treatment are therefore increased. In addition, these aesthetical, functional, and long-term results are obtained by a method which is simple and efficient in its application.

As it will be explained later, the determined performance of the jaw function may correspond to the performance considered satisfactory according to bibliographic data, or to the performance that it considered satisfactory considering the physiology of the individual's jaw.

In this text, the term "individual" refers to the one who is intended to receive the planned dental treatment. The individual is for example a person. Alternatively, the individual is any other animal provided with teeth.

According to different aspects, it is possible to provide the one and/or the other of the characteristics below taken alone or in combination.

According to one embodiment, the method may further comprise determining an actual performance of the jaw function of the individual, the determined performance on which the wax-up is based corresponding to said actual performance or to a modified performance of the jaw function of the individual with respect to said actual performance. In this disclosure, the expression "actual performance" refers to the performance of the individual's jaw function before receiving the planned dental treatment. The determination of the actual performance of the jaw function allows to determine if, before the dental treatment, the jaw function of the individual is already satisfactory or not. If the actual performance of the jaw function is already satisfactory, the automated wax-up production may be based directly on this actual performance. On the contrary, if the actual performance of the jaw function is not completely satisfactory, the wax-up may be based on the modified performance of the jaw function. This modified performance corresponds advantageously to a satisfactory performance of the jaw function according to the jaw's physiology of the individual. The modified performance corresponds in another embodiment to a satisfactory performance of the jaw function according to bibliographic data.

In the present disclosure, the expression "wax-up based on X performance" means that the wax-up proposes a position and/or shape of the individual's intraoral structures that ensures that at the end of the planned dental treatment, the jaw function of the individual will perform according to the X performance, X being "actual", "determined" or "modified". For example, if the wax-up is based on the actual performance of the jaw function of the individual, then at the end of the planned dental treatment the jaw function will perform as it did before the application of the treatment. If the wax-up is based on a modified performance of the individual's jaw function, at the end of the planned dental treatment the performance of the jaw function will be different to how it was before the treatment, in particular it will perform according to the modifications provided by the wax-up. As it will be detailed later, the performance of the jaw function depends on parameters such as mandible position, and/or anterior guidance and/or occlusal plane. The mandible movement may also have an impact on the performance of the jaw function. When this disclosure refers to the "modified performance", this means that at least one of these parameters is changed compared to their value/state in the actual performance of the individual's jaw.

Furthermore, in the context of the present disclosure, the wax up may be obtained based on an occlusal plane determined from the mandibular position and the anterior guidance. The occlusal plane interdependent with these parameters constitutes the basis for all adjustments performed by the automated wax up generation process. Accordingly, the proposed position and/or shape of the intraoral structures is constructed or adapted in accordance with the occlusal plane derived from the mandibular position and the anterior guidance, ensuring that the planned dental treatment remains consistent with performance of the jaw function of the individual's jaw.

According to one embodiment, the method may further comprise registering an image of at least part of the individual's jaw, preferably at least part of the jaw in movement. The image is for example registered before determining the actual performance of the jaw function, as said image may be used to determine the actual performance of the jaw function, as it will be detailed later. The image may also be registered before digitally and automatically obtaining the wax-up built based on the determined performance of the jaw function. When the determined performance of the wax-up is based on the performance considered satisfactory according to bibliographic data, the image may also be registered during or after the wax-up is obtained. In that case, the image may be useful to obtain a second wax-up showing the current state of the intraoral structures in the individual to be treated. This second wax-up may then be superimposed to the wax-up built based on said determined performance of the jaw function, which may help the professional to plan the gestures and manipulations that are to be applied to the individual's jaw during the dental treatment. **It** should be noted that such a second wax-up showing the current state of the intraoral structures may be obtained and superimposed to the wax-up built based on said determined performance of the jaw function in any of the embodiments described in the present text or their variations, regardless of whether said determined performance corresponds to a performance based on bibliographic data or on a performance based on the physiology of the individual's jaw.

According to one embodiment, determining the actual performance of the jaw function may include obtaining a diagram of the jaw's movement. Such a diagram helps the professional and/or an artificial intelligence module that is/are planning the dental treatment to decide whether the individual's jaw moves satisfactorily or whether it can be improved. As it will be detailed, the diagram of the jaw's movement may be obtained after having analysed:
- an opening and/or closing trajectory of the individual's mandible, and/or
- a dynamic occlusion of the individual's mandible and maxilla.

In this disclosure, the expression "dynamic occlusion" refers to the way the teeth come into contact with each other during mandible's movements. It focuses on how the upper and lower teeth contact each other when the mouth, and mainly the mandible, is in motion. The dynamic occlusion may therefore be analyzed by examining a right-left laterotrusion trajectory of the individual's mandible and/or a propulsion trajectory of the individual's mandible, among other movements.

According to one embodiment, determining the actual performance of the jaw function may include determining a mandible position and/or a mandible movement of the individual. Determining the mandible position and/or the mandible movement allow(s) determining the position of the temporomandibular joint (also known as TMJ).

According to one embodiment, the mandible position may be determined by analysing:
- an opening and/or closing trajectory of the individual's mandible; and/or
- a dynamic occlusion of the individual's mandible and maxilla; and/or
- a static occlusion of the individual's mandible and maxilla.

The mandible movement may be determined by analysing:
- an opening and/or closing trajectory of the individual's mandible; and/or
- a dynamic occlusion of the individual's mandible and maxilla.

The mandible position and/or the mandible movement is/are therefore determined by analysing easily accessible parameters.

According to one embodiment, determining the actual performance of the jaw function of the individual may comprise determining anterior guidance.

According to one embodiment, the anterior guidance may comprise obtaining an overjet value and/or an overbite value of the individual's jaw. The anterior guidance may also comprise determining the position of the six upper front teeth.

According to one embodiment, determining the actual performance of the jaw function of the individual may comprise determining an occlusal plane.

According to one embodiment, the wax-up may include a corrected functional shield of the individual's mandible and/or a corrected mandible position and/or a corrected teeth position when the actual performance of the jaw function is unsatisfactory.

In this disclosure, the expression "functional shield" refers to the space in which the dental arches can move freely, without interference, while ensuring efficient jaw function and a stable TMJ. In the case of human anatomy, there are two dental arches: the maxillary arch, which includes all of the individual's upper teeth, and the mandibular arch, which includes all of the individual's lower teeth.

According to one embodiment, at least an artificial intelligence model may be employed for digitally and automatically obtaining the wax-up. The wax-up is therefore obtained easily, quickly and with a high degree of precision.

According to one embodiment, at least an artificial intelligence model may be employed for digitally and automatically creating a virtual articulator adapted to simulate and/or correct the jaw function of the patient.

According to one embodiment, at least an artificial intelligence model may be employed for determining the actual performance of the jaw function. The actual precision is therefore determined easily, quickly and with a high degree of precision.

According to one embodiment, the wax-up may simulate all individual's intraoral structures or a part of the individual's intraoral structures. The wax-up is therefore adapted to the extent of the planned treatment.

According to one embodiment, the method may further comprise milling or printing a dental prothesis, and/or a dental orthosis, and/or orthodontic devices, and/or guides, and/or mock-ups considering the position and/or shape of the intraoral structures in the wax-up. The milled/printed structures correspond therefore to the structures proposed by the wax-up in shape so that their installation in the individual's jaw in the position proposed by the obtained wax-up is facilitated.

According to one embodiment, it is also proposed an installation for implementing the method described above, the installation comprising a wax-up generation module configured to digitally and automatically modelling the proposed position and/or shape of individual's intraoral structures, the wax-up generation module being configured to take into account said determined performance of the jaw function when modelling said wax-up.

According to one embodiment, the installation may further comprise a jaw function module configured to determine an actual performance of the jaw function of the individual.

According to one embodiment, the installation may further comprise a jaw tracker module configured to obtain a 3D model of individual's jaw dynamics from analysing an image of at least part of the jaw of the individual.

The jaw tracker module is software-based and adapted to avoid the use of artificial markers or additional hardware.

According to one embodiment, said 3D model of the individual's jaw dynamics is combined, by the jaw function module, with intraoral scan data so as to generate a functional and dynamic digital model of the entire jaw. This representation does not merely reproduce the static and dynamic occlusion of the jaws but provides a comprehensive functional and dynamic digital model of the entire jaw.

According to one embodiment, the wax-up is generated digitally and automatically from the dynamic and functional constraints derived from the functional and dynamic digital model of the entire jaw based on the actual or on a modified performance of the jaw function that corresponds to a satisfactory performance of the jaw function according to the jaw's physiology of the individual or, alternatively, according to bibliographic data.

According to another embodiment, an existing digital wax-up model can be analyzed and, if necessary, modified and/or improved in terms of jaw function and/or aesthetics according to bibliographic data and/or patient preferences and/or professional's opinion.

According to one embodiment, the installation may further comprise a milling and/or printing module configured to mill and/or print a dental prothesis, and/or a dental orthosis, and/or orthodontic devices, and/or guides, and/or mock-ups considering the position and/or shape of the intraoral structures in the wax-up.

According to one embodiment, it is also proposed a computer program comprising instructions which, when the program is executed by a computer, cause the computer to carry out the method described above. The computer program may comprise an artificial intelligence model, for example a machine learning model or data set.

The expression "dental treatment" refers to the various care provided by a dentist or dental professional to maintain, restore, or improve oral health. This can include a wide range of services, such as:
- preventive care; and/or
- restorative procedures: fillings, crowns, and/or bridges to repair damaged or missing teeth; and/or
- orthodontics: braces, aligners, and other devices to correct misaligned teeth and jaws; and/or
- periodontics: treatment of gum disease and other conditions affecting the supporting structures of the teeth; and/or
- endodontics: root canal therapy to treat infections inside the tooth; and/or
- prosthodontics: creation of dentures and other prosthetic devices to replace missing teeth and/or
- biomechanical analysis: implants to repair damaged or missing teeth; and/or
- cosmetic dentistry: procedures like teeth whitening, veneers, and bonding to enhance the appearance of teeth.

In the present text, "jaw" refers to the lower part of the face that includes the bones (namely the mandible and the maxilla), muscles, teeth, skin and any other intraoral structure, whether anatomical or artificial. It is primarily responsible for the movement and function of the mouth, such as chewing and speaking and smiling.

The expression "intraoral structures" include in this disclosure every anatomical or artificial structure found within the individual's mouth. Examples of anatomical intraoral structures are the teeth, the gums, the tongue, the palate, the cheeks, etc. Artificial intraoral structures are synthetic devices and/or materials used to replace or support anatomical structures within the mouth, in order to restore function, improve aesthetics and/or maintain oral health. Examples of artificial intraoral structures are dental implants, dentures, crowns, bridges, orthodontic appliances, elastodontic appliances, etc.

In this disclosure, the terms 'digitally' and 'automatically' refer to actions, such as calculations and/or modelling, among others, carried out by a machine. The human intervention is mainly limited to starting the machine and/or entering data. As will be detailed, data can be entered via an image that can be analysed by the machine to extract more data. The machine may be a computation machine, for example a computer, without this being restrictive.

"Wax-up" refers to a detailed model of a patient's teeth crafted to represent the desired final outcome of a dental treatment, such as crowns, or any other temporary or final prosthesis. It serves as a valuable analysis and planning tool, allowing both the dentist and the patient to visualize the proposed changes and ensure that the treatment plan is achievable and meets the patient's expectations.

In the present disclosure, the term "shape" refers not only to the geometrics of each intraoral structure, but also to its size.

In this text, the expressions 'maxillary teeth' and 'upper teeth' are used interchangeably. The expressions 'mandibular teeth' and 'lower teeth' are also used interchangeably in this disclosure.

### BRIEF DESCRIPTION OF THE DRAWINGS

[Fig. 1] schematically represents a method for planning a dental treatment on an individual according to a first embodiment.
[Fig. 2] schematically represents a method for planning a dental treatment on an individual according to a second embodiment.
[Fig. 3] schematically represents different actions that may be included in a step of determining an actual performance of the jaw function of the individual of the method of Figures 2 and 6.
[Fig. 4] schematically represents a detail of a mandible on which the method of Figures 1, 2, 5 and 6 may be applied.
[Fig. 5] schematically represents a method for planning a dental treatment on an individual according to a third embodiment.
[Fig. 6] schematically represents a method for planning a dental treatment on an individual according to a fourth embodiment.
[Fig. 7] schematically represents an installation for implementing the method of the previous Figures according to a first embodiment.
[Fig. 8] schematically represents an installation for implementing the method of the previous Figures according to a second embodiment.
[Figure 9A] represents a first diagram of the jaw's movement obtained by the method of the previous Figures.
[Figure 9B] represents a second diagram of the jaw's movement obtained by the method of the previous Figures.
[Fig. 10] schematically represents an example of an installation for implementing the method of the previous Figures according to a third embodiment.
[Fig. 11] schematically represents a computer program comprising instructions which, when the program is executed by a computer, cause the computer to carry out the method of the previous Figures.

In the drawings, identical references designate identical or similar objects.

### DESCRIPTION OF EMBODIMENTS

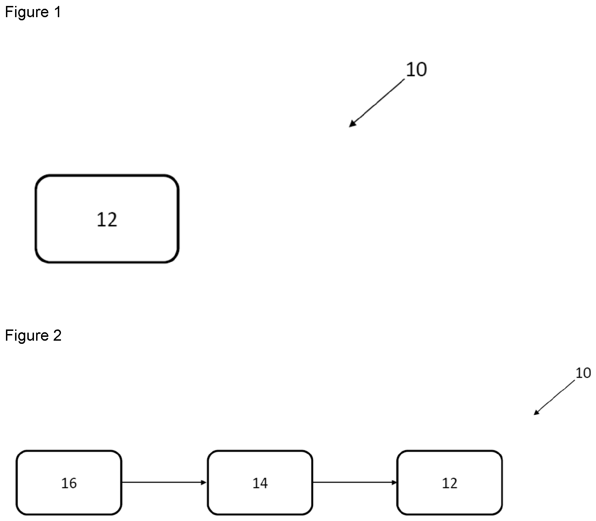

A method 10 for planning a dental treatment on an individual according to this disclosure is now described with reference to figures 1 to 6.

As shown in figure 1, the method 10 comprises a step 12 comprising digitally and automatically obtaining a wax-up built based on a determined performance of the jaw function of the individual. This wax-up is a digital model, preferably a 3D model, of the intended final outcome of the planned dental treatment. In particular, the wax-up shows a proposed position and/or shape of the individual's intraoral structures, for example the teeth and the artificial intraoral structures that are installed and/or intended to be installed in the individual's mouth. In some cases, such as implantology, the wax-up allows the position of the implants in the bone to be visualised, as well as the surgical reconstructions of the bone or gum, which are convenient for better biomechanics and aesthetics.

The wax-up may simulate all individual's intraoral structures. In other cases, the wax-up simulates only a part of the individual's intraoral structures. It is even possible that the wax-up simulates only an intraoral structure of the individual, for example one tooth. In particular, depending on the extent of the planned dental treatment, the wax-up simulates the whole, some or only one individual's intraoral structure(s).

In this context, the method enables treatment planning that is limited to a tooth, a single dental arch, extends to both the maxillary and mandibular arches, or encompasses the full dentition.

Advantageously, simulating the individual's intraoral structure(s) means simulating the static and dynamic occlusion of the jaws but also the entire function of the jaws.

The wax-up may be obtained thanks to a wax-up generation module 52 that will be detailed below. Advantageously, at least an artificial intelligence model is employed for digitally and automatically obtaining the wax-up based on the determined performance of the jaw function. An example of artificial intelligence model for obtaining the wax-up will also be explained later.

In one embodiment, the determined performance of the jaw function corresponds to the performance considered satisfactory according to bibliographic data based on, for example, the age, sex, race, etc. of the individual. The determined performance may take into account, among others, the following parameters of the jaw function:
- a mandible position, and/or
- a mandible movement, and/or
- an anterior guidance, and/or
- an occlusal plane.

When the determined performance corresponds to the performance which is considered satisfactory in the bibliography, the values of these parameters correspond also to the values that are considered satisfactory in the bibliography for each of them. For example, with respect to the mandible position, bibliographic data considers that a myocentric position of the mandible is satisfactory. The "myocentric position" refers to the position of the jaw where the muscles of the mandible are at their most relaxed, requiring minimal energy to bring the teeth into proper alignment or intercuspation.

In a favorable example, it is a professional who considers whether the performances are satisfactory.

In an alternative embodiment of the method 10 represented by figure 2, a step 14 comprising determining an actual performance of the jaw function of the individual is carried out before obtaining the wax-up according to step 12. The expression "actual performance" corresponds here to the real performance of the jaw function of the individual. **In** other words, step 14 comprises conducting a study of the function of the individual's jaw to determine whether the individual's jaw is functioning completely satisfactorily prior to the planned treatment or whether there is room for improvement.

As it will be detailed below, for implementing step 14, a step 16 comprising registering at least an image of at least part of the individual's jaw may be carried out beforehand.

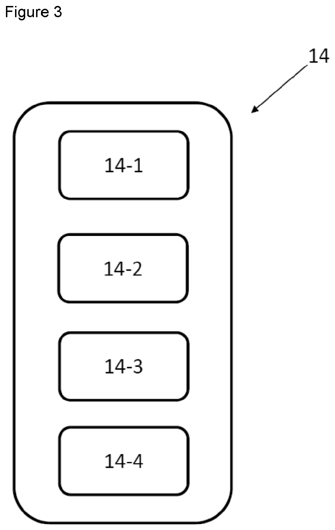

The determination 14 of the actual performance of the jaw function comprises one or several of the actions 14-1 to 14-4 that are represented in figure 3. It must be noted that the order of description of each of these actions 14-1 to 14-4 does not mean that these actions must be carried out in such an order to implement the method 10. On the contrary, these actions 14-1, 14-2, 14-3, 14-4 may be implemented in any order, and, as already said, it is not compulsory that all of them are carried out.

The determination 14 of the actual performance of the jaw function comprises for example determining 14-1 a mandible position of the individual. The mandible position may be determined by analysing the jaw motion and/or the teeth position, namely the cuspid of the teeth, which is a guiding element in the mastication. To that end, images of the individual's jaw are taken (step 16 on the diagram of figure 2). Of course, the registered images may show partially the individual's jaw. For example, the registered images may show only part of the individual's teeth. In other cases, every tooth may be visible on the registered images.

Advantageously, while images are taken during step 16, the individual's jaw, and particularly the individual's mandible, is in motion. In order to take such images, any kind of device able to take pictures and/or videos can be used. For example, the images may be taken with a camera or with a mobile phone provided with a camera. Alternatively, or complementarily, an intraoral scan or any other imaging technique respecting the DICOM (Digital Imaging and Communication in Medicine) standard may be employed to obtain these images of the individual's jaw.

For example, the images taken during step 16 are images of the jaw from the face side.

In some cases, as it will be detailed below, one or more markers are attached to the individual's jaw while the images of the jaw are being taken. Such markers may be attached for example to one tooth or several teeth, and/or to the gums, and/or to the chin, and/or to the lips, etc. The individual may wear advantageously a lip retractor during imaging in order to see all the teeth on the taken images.

In other cases, as it will be detailed below, there is no artificial marker attached to one or more teeth, gums, chin, and/or lips, and the jaw tracker module 56 is then provided with an artificial intelligence model adapted to recognize anatomical features.

The movements of the jaw while images are being taken include mainly movements that activate the temporomandibular joint (also known as TMJ). These movements may thus include:
- an opening and/or closing trajectory of the individual's mandible; and/or
- a dynamic occlusion of the individual's mandible and maxilla.

The dynamic occlusion of the individual's mandible and maxilla includes in particular successive movements of laterotrusion right and of laterotrusion left, and/or propulsion movements of the individual's mandible. This allows the individual's functional manducatory angles to be determined.

In addition, images of the individual's jaw may also be taken during static occlusion of the individual's mandible and maxilla. Static occlusion allows determining, for example, if problematic interferences between mandibular teeth and maxillary teeth exist in the individual's jaw.

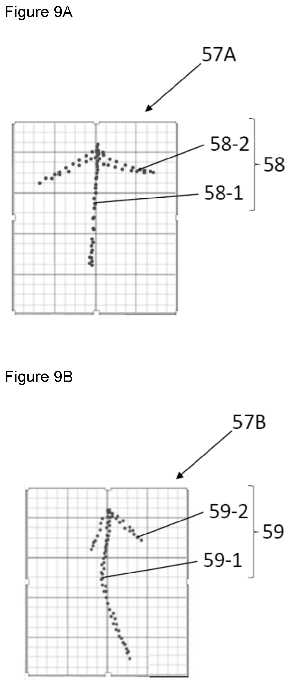

As it will be described later, all the images taken of the individual's jaw are advantageously treated by a jaw tracker module 56 which may be able to recognize the teeth (or markers) and match them to the previously recorded digital impressions of the individual's mandible and maxilla. A diagram 50 of the jaw's movement, which will be described later with reference to figures 9A and 9B, may be obtained when the images registered during step 16 are treated by the jaw tracker module 56.

By "recognize the teeth", it is understood that the jaw tracker module 56 recognizes teeth that do not bear artificial markers.

Tracking of jaw movements is traditionally performed by a dedicated hardware system comprising sensors, artificial markers, or mechanical devices attached to the jaw to directly measure its dynamics. In contrast, the jaw tracker module 56 is implemented as a software module that processes image sequences acquired by an image capture device that will be described later.

The jaw tracker module 56 identifies visible features (teeth or markers), matches them to previously recorded digital impressions of the jaw, and reconstructs the jaw dynamics from successive images. The software processing yields a diagram 50 representing the jaw movement, without resorting to specialized tracking hardware.

According to one embodiment, the jaw tracker module 56 includes an artificial intelligence model adapted to process images, recognize teeth and match them to previously intraoral scans. For example, such diagrams help the professional to assess whether the patient's jaw movement is satisfactory or whether it can be improved.

In addition, the images recorded are advantageously analysed by a jaw function module 54 that will be described below. For example, the jaw function module 54 analyses the images after having been treated by the jaw tracker module 56. Therefore, mandible position is advantageously determined by analysing:
- an opening and/or closing trajectory of the individual's mandible; and/or
- a dynamic occlusion of the individual's mandible and maxilla; and/or
- a static occlusion of the individual's mandible and maxilla.

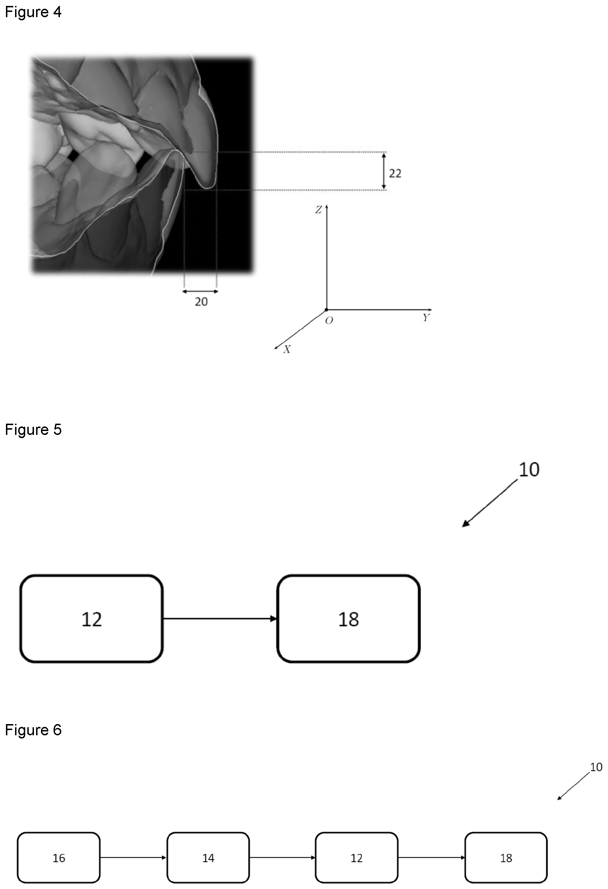

The determination 14 of the performance of the jaw function of the individual may comprise determining 14-2 anterior guidance. Anterior guidance is defined by the position of the social six (i.e. the six upper front teeth), by overjet value and by overbite value. Determining 14-2 anterior guidance may therefore comprise obtaining an overjet value and/or an overbite value. As shown in figure 4, overjet 20 corresponds to the horizontal gap between the upper and lower teeth of the individual, while overbite 22 corresponds to the vertical overlap of the upper teeth over the lower teeth of the individual.

A satisfactory anterior guidance may exist when there is sufficient functional sagittal clearance between the upper and lower teeth to allow forward movement of the lower teeth. However, in order to ensure a satisfactory function of the individual's jaw, excessive values of overjet 20 and overbite 22 should be avoided. Generally, overjet 20 and overbite 22 values greater than 3 mm are considered excessive.

As it will be described later, overjet 20 and overbite 22 value may be determined using the Slavicek triangle, but this is not restrictive.

The determination 14 of the jaw function may comprise determining 14-3 an occlusal plane of the individual's jaw. The occlusal plane is a virtual surface formed by the occlusal (biting) surfaces of the teeth when the jaw is closed. Essentially, it represents the average curvature of these surfaces rather than a flat plane.

As it will be described later, the occlusal plane of the individual's jaw may be determined by applying different methods, such as for example the Cooperman Plane or the Monson sphere.

According to one embodiment, it is the jaw function module 54 that can determine the occlusal plane of the individual jaw using at least one of the Cooperman planes and the Monson sphere.

The anterior guidance parameters (i.e. the position of the six social and the values of overjet 20 and overbite 22) and the occlusal plane of the individual to be treated can also be determined thanks to the jaw function module 54 as it will be described below.

The determination 14 of the actual performance of the jaw function may further comprise determining 14-4 a mandible movement of the individual. To that end, the images taken during step 16 may be analysed, namely those taken while the mandible is in motion. In particular, it is possible to determine the mandible movement of the individual by analysing:
- an opening and/or closing trajectory of the individual's mandible; and /or
- a dynamic occlusion of the individual's mandible and maxilla.

As for determining 14-1 the mandible position, the images taken of the individual's jaw are advantageously treated by the jaw tracker module 56 which may be able to recognized the teeth and match them to the previously recorded digital impressions of the individual's mandible and maxilla.

In one embodiment, the intraoral scan is a conventional digital scan of the inside of the mouth. It is configured to capture digital impressions of the maxillary and mandibular arches, the individual's soft tissues, and the occlusal relationship, for example in the form of a high-resolution 3D mesh typically provided as an STL, PLY or XML file.

The intraoral scan corresponds to a 3D digital acquisition obtained with a handheld optical scanner operating by projecting a light beam, such as structured light and/or a laser.

The acquisition itself is static: the scanner captures successive frames that are stitched together by the software to reconstruct a still 3D model. This model provides an accurate and measurable representation of the intraoral situation at a given point in time, usable as a geometric reference for all subsequent diagnostic, prosthetic, and manufacturing steps, for example, as a reference for the static occlusal position.

According to a preferred embodiment, the jaw movements observed on the diagrams are deemed satisfactory by the professional, who can then directly generate a wax-up based on these performances with the wax-up generation module 52, without following the steps described below in the jaw function module 54. The professional must then validate the proposed wax-up.

In the event that the wax-up is not validated by the professional, the latter may make manual and/or automatic modifications, the jaw tracker module 56 will then trace the diagrams of the new jaw and the professional will have to determine again whether the movements are satisfactory or not.

If the jaw movements are not deemed satisfactory on the diagrams plotted by the jaw tracker module 56 then the jaw performance must be modified by the jaw function module 54, as detailed below.

In addition, the images recorded are advantageously analysed by the jaw function module 54 that will be described below. For example, the jaw function module 54 analyses the images after having been treated by the jaw tracker module 56.

The jaw function module 54 is designed to generate a functional and dynamic digital model of the entire jaw.

Said functional and dynamic digital model of the entire jaw is adapted to correct an existing wax-up, customizing its shape to the patient's exact jaw function or the patient's corrected jaw function.

In one embodiment, the images taken during step 16 cooperate with the intraoral scan.

The 3D scan obtained with the intraoral scan and the 2D photos and/or videos obtained from the images are registered using a smile design function.

According to one example, the smile design function can be included in the jaw function module 54 or performed by a separate device.

In one example, the smile design function uses markers identified in both datasets. These markers may consist of anatomical landmarks, such as incisal edges or cusp tips, or reference stickers affixed to teeth or soft tissues.

The system smile design function then performs a 2D-3D registration. The facial and dental landmarks detected on each image are matched with their corresponding points on the 3D mesh.

In the case where the images include at least one video, the facial and dental landmarks detected on each video frame are matched with their corresponding points on the 3D mesh.

Said registration allows the static intraoral scan to be overlaid in real time onto the moving video, thereby generating a functional and dynamic digital model of the entire jaw.

The maxilla is automatically positioned, by the jaw function module 54, in the frame of reference of said functional and dynamic digital model of the entire jaw by using the Camper plane.

The positioning of the maxillary and mandibular arches is performed automatically. The mandibular arch is then positioned in relation to the maxillary arch.

The Camper plane is a cranio-facial anatomical reference plane defined by three landmarks that are identifiable from the outside of the patient's face. Said landmarks comprise the subnasal point, located at the base of the nasal septum, as well as the right tragus and the left tragus. These three points, which are readily locatable by visual inspection and/or by algorithmic detection, define a plane that is substantially parallel to the dental occlusal plane in the majority of patients.

According to one exemplary embodiment, the Camper plane is identified from one or more images taken during step 16, on which the aforementioned three landmarks are detected, either manually by the operator or, advantageously, automatically by means of a markers detection algorithm of the jaw tracker module 56.

According to another exemplary embodiment, said Camper plane is identified using the intraoral scan and its 3D mesh, the landmarks being extracted from the acquired three-dimensional data.

Alternatively, the intraoral scan can be replaced by a cone beam computed tomography (CBCT).

According to a further exemplary embodiment, the two data sources, namely the images taken during step 16 and the 3D mesh resulting from the intraoral scan, are combined, the Camper plane then being identified on the patient-specific 4D representation.

According to one embodiment, the occlusal plane is aligned substantially parallel to the previously identified Camper plane. The maxilla is oriented within the reference frame of the 3D model generated by the jaw tracker, according to the occlusal plane determined by the Camper plane. Said positioning enables the maxilla to be located in an anatomical reference frame consistent with the recorded mandibular dynamics, without requiring the use of a physical facial bow or any hardware devices.

The HIP plane is an intraoral anatomical reference plane defined by three landmarks located on the palatal structures of the patient. Said landmarks comprise the incisive papilla, as well as the right pterygoid hamulus and the left pterygoid hamulus.

When the individual was previously submitted to imaging, such as an intraoral scan or a computed tomography, said anatomical landmarks are identified on intraoral scans and/or cone-beam computed tomography (CBCT) data by the smile design function, and placed in the frame of reference of said functional and dynamic digital model of the entire jaw.

For example, anatomical landmarks include gingival tissues. Indeed, an alteration or omission of gingival tissues would compromise the detection of anatomical landmarks necessary for an accurate recording.

According to one embodiment, the three aforementioned landmarks, namely the incisive papilla and the two pterygoid hamuli, are extracted directly from the acquired three-dimensional data, either manually by the operator by means of a selection interface, or advantageously automatically by means of a three-dimensional landmark detection algorithm applied to the 3D mesh of the intraoral scans and/or cone-beam computed tomography (CBCT).

Obtaining the HIP plane enables the occlusal plane to be oriented and thereby allows the maxilla to be automatically positioned within the reference frame of the functional and dynamic digital model of the entire jaw generated by the jaw function module 54.

The impressions of the maxillary and mandibular arches are repositioned in a unified and normalized 3D coordinate system to ensure the consistency of the calculations.

According to one example, the quality of the recording of said arches is validated by a quantitative measurement of the inter-surface distances, with a maximum permissible tolerance of 0.1 mm.

In one embodiment, the jaw function module 54 includes an algorithm adapted to detect abnormal interpenetration zones between occlusal surfaces and apply an initial vertical translation to minimize overlaps.

In one embodiment, the wax-up generation module 52, that will be detailed below, includes an algorithm adapted to align the wax-up to the corresponding arch using an algorithm that detects characteristic points located on gingival tissues.

The said algorithm is adapted to identify anatomical landmarks and establish a correspondence between the proposed prosthetic model and the patient's actual anatomy, including the patient's gingival structures in their natural and unmodified state.

In one embodiment, the three-dimensional dynamic model of the jaw, obtained by intraoral scans, is combined, by the smile design function, with images (photos/videos) taken during step 16 to obtain a functional and dynamic digital model of the entire jaw.

Said functional and dynamic digital model of the entire jaw is not only a digital model of the static and dynamic occlusions of the jaw but also a digital model of the entire function of the jaw.

As an example, intraoral scanning data is retrieved beforehand.

Said functional and dynamic digital model of the entire jaw incorporate a virtual physiological articulator adapted so that the virtual mandible can move on the virtual maxilla.

As an example, the virtual articulator can also be described as a virtual avatar or digital twin.

In one embodiment, the jaw function module 54 also includes a dynamic simulation module which includes the virtual physiological articulator.

The dynamic simulation module reproduces natural mandibular movements based on said virtual articulator and automatically-configured age-dependent parameters.

Said virtual articulator is configured to be specifically adjusted, by means of mathematical computations, for full-arch reconstructions, so as to meet the functional requirements of the individual being treated, in accordance with jaw tracking data obtained by the jaw tracker module 56 that will be described later.

According to one embodiment, the virtual articulator is adapted to simulate at least one of the following movements:
- protrusion;
- retrusion;
- opening;
- lateral movements (right/left, including Bennett's movement).

During the simulation of said mandibular movements, the system is further configured to detect dental collisions, including premature contacts and functional interferences. A distance calculation algorithm quantifies any interpenetration zones with a detection accuracy below 0.1 mm. The identified interferences are subsequently used as input data for automated occlusal correction processes.

In an embodiment, the jaw function module 54 is adapted to automatically position the Monson sphere in the model it created.

Said Monson sphere is a geometric reference surface according to which the occlusal surfaces of the posterior teeth are inscribed on a portion of a sphere whose center is located at the glabella of the patient. It is adapted to have a radius substantially equal to 100 mm.

Said Monson sphere serves as a three-dimensional geometric reference for automatically positioning the cusps and incisal edges of the reconstructed dental arch along an ideal occlusal curvature.

In one embodiment, the curvature of the occlusal plane is adapted to the Monson sphere to maximize their contact surface with a tolerance predetermined.

In one embodiment, the jaw function module 54 also includes an optimization function.

According to one example, the optimization function involves an algorithm.

The optimization function is suitable to minimize the sum of radial distances between the Monson sphere and the sampled occlusal points of the occlusal plane whose shape is determined with the Monson sphere.

The optimization function is suitable to maximize the number of points between the Monson sphere and the occlusal plane at a distance that respects the predetermined tolerance.

The optimization function is suitable to increase weighting on functional cusps and marginal ridges to preserve functional geometry.

In one embodiment, the jaw function module 54 also includes an aberrant teeth detection function.

According to one example, the aberrant teeth detection function involves an algorithm.

The function is adapted to analyze each tooth to detect those whose position or orientation deviates significantly from the average position and impairs functional contacts of the occlusal plane.

The analysis of each tooth includes measuring in millimeter the deviation from the average sphere and/or measuring the contact density corresponding to the proportion of the occlusal surface in contact within the tolerance limits.

The analysis of each tooth includes aberration detection criteria such as irregular height if the average deviation is greater than a predetermined threshold 0.30 mm and/or abnormal contact if density falls below a low predetermined thresold < 20% (insufficient) or exceeds a high predetermined threshold> 85% concentrated on a small area (localized overload).

If one of the measurements corresponds to one of the criteria, then the tooth is considered aberrant.

By determining at least one among the mandible position, the mandible movement, the anterior guidance and the occlusal plane of the individual's jaw, the present method allows determining 14 the actual performance of the jaw function. In particular, as it will be detailed, the jaw function module 54 is provided with an artificial intelligence module which is able to determine 14 the actual performance of the jaw function of the individual when at least one among the mandible position, the mandible movement, the anterior guidance and the occlusal plane of the individual's jaw is/are known.

In some cases, the actual performance of the jaw function is satisfactory. In these cases, advantageously, the determined performance on which the wax-up is based corresponds to the actual performance of the individual's jaw function.

In other cases, the actual performance of the jaw function may present features that may be improved to provide the individual with a better performance of the jaw function. Examples areas for improvement may be incorrect dental alignment, lost teeth, malocclusion, jaw dislocation, etc. In these cases, the wax-up is advantageously based on the modified performance of the jaw function that corresponds to a satisfactory performance of the jaw function according to the jaw's physiology of the individual or, alternatively, according to bibliographic data. In particular, the wax-up includes a corrected functional shield of the individual's mandible and/or a corrected mandible position and/or a corrected teeth position when the actual performance of the jaw function is unsatisfactory.

According to one embodiment, when the actual performance of the jaw function presents features that may be improved and/or are deemed unsatisfactory, the jaw function module 54 proposes a new jaw function, the performance of which is evaluated to determine whether it is satisfactory and can therefore be validated. If the performance remains insufficient, the jaw function module 54 continues to propose successive improvements. Once a satisfactory version is obtained, it is retained as the basis for the wax up.

According to one embodiment, the evaluation of the performance of the new jaw function proposed by the jaw function module 54 is carried out as follows. A modified functional and dynamic digital model of the entire jaw is generated from the initial functional and dynamic digital model of the entire jaw or from a previous modified functional and dynamic digital model of the entire jaw, in which only those anatomical elements modifiable through restorative and/or orthodontic intervention are altered.

According an exemple, the elements considered immutable, in particular the shape of the mandible and of the maxilla, are preserved unchanged.

The elements considered modifiable, such as the occlusal morphology of the teeth, the incisal edges, the position and orientation of one or more teeth within a physiologically and orthodontically achievable range, could be adjusted.

The virtual articulator is then applied to said modified functional and dynamic digital model of the entire jaw in order to verify that the jaw function is now satisfactory.

If the modifications result in a satisfactory jaw function, then the corresponding criteria are met, the new jaw function is validated and retained as the basis for the wax-up model. Otherwise, the jaw function module 54 iteratively modifies one or more of these modifiable elements and repeats the simulation on the updated model until satisfactory performance is achieved.

According to one embodiment, when the jaw function is validated, its wax-up is generated by the wax-up generation module 52 so that the professional can validate.

If the wax-up is not approved, the professional can make adjustments using the wax-up generation module 52; the modified wax-up is undergoing again the jaw movements in the virtual articulator and is automatically corrected to fit the jaw function of the patient.

In one embodiment, the jaw function module 54 also includes an adjustment proposals function adapted to propose improvements.

According to an example, the adjustment proposals function involves an algorithm adapted to make adjustment proposals when one or more teeth are considered as aberrant.

According to an example, the wax-up in question is a wax-up, whose jaw function, on which it is based, is satisfactory or not, external to the system imported as an STL file.

According to this example, the adjustment proposals function evaluates one or more corrective actions among predefined categories of adjustments. The corrective actions may include:
- Material addition (local build-up) when the contact density is below a predetermined threshold and/or when the tooth presents a negative deviation exceeding a given limit. In this case, the algorithm proposes a local build-up ranging from a first predetermined value to a second predetermined value, followed by a smoothing operation to ensure morphological continuity.
- Material removal (local reduction) when a local excess of material is detected and/or when a simulated occlusal interference is identified. In such cases, the algorithm proposes a local reduction ranging from a first predetermined value to a second predetermined value, followed by smoothing to restore a coherent surface geometry.

Global vertical displacement when the average deviation of the tooth exceeds a predetermined threshold while maintaining consistent overall geometry. The algorithm then proposes incremental vertical adjustments combined with interproximal control and repeated simulation of the jaw function to validate the correction.

In one example, the version selected for the wax-up is exported as an STL file with a digital report detailing the modifications done by the jaw function module 54, as it will be detailed later.

The wax-up is generated digitally and automatically from the dynamic and functional constraints derived from the functional and dynamic digital model of the entire jaw based on the actual or on a modified performance of the jaw function that corresponds to a satisfactory performance of the jaw function according to the jaw's physiology of the individual or, alternatively, according to bibliographic data.

In one embodiment, the performance of jaw function is evaluated using the physiological virtual articulator in which the mandible moves on the maxilla.

It should be noted that the step 16 of registering images of at least part of the individual's jaw may also be used to obtain a second wax-up that shows the current state of the intraoral structures in the individual to be treated. This second wax-up can then be superimposed to the wax-up built based on the determined performance of the jaw function, which may help the professional to plan the gestures and manipulations that are to be applied to the individual's jaw during the dental treatment. This means that, when the obtained wax-up is based on a performance that is satisfactory according to the bibliography, the step 16 comprising the registration of images may be done before, during or after the wax-up is obtained, because the second wax-up can be determined at any moment before the application of the dental treatment.

In a non-limiting embodiment, said images can be a set of one or more photographs and/or one or more videos.

As an example, the videos can then be cut into several images.

The images are characterized to model the complete kinematics and/or dynamics of the jaw.

The images are taken with an image capture device.

Examples of image capture devices are smartphone, tablet, digital camera, webcam, video camera, laptop camera, dental X-ray machine, Scanner (CT scan), dental camera, intraoral camera, endoscope.

As shown in figures 5 and 6, the method 10 may comprise a step 18 comprising milling or printing a dental prothesis, and/or a dental orthosis, and/or orthodontic devices, and/or guides, and/or mock-ups considering the position and/or shape of the intraoral structures in the obtained wax-up.

"Dental prosthesis" comprise artificial devices used to replace or repair missing or damaged teeth and other structures within the mouth. Examples of dental prothesis are crowns, bridges, dentures, implants, veneers, etc.

"Dental orthosis" refers in this disclosure to custom-made appliance designed to correct or manage various dental and jaw-related issues. Examples of dental orthosis include braces, aligners, retainers, mouthguards, splints, palatal expanders, etc.

"Orthodontic devices" comprise tools used to correct dental misalignments and improve the function and appearance of the teeth and jaw. Some orthodontic devices are also dental orthosis. Examples of orthodontic devices include braces, aligners, mouthguards, retainers, palatal expanders, headgears, space maintainers, lip and cheek bumpers, etc.

"Mock-up" refers to diagnostic and planning tools used in cosmetic and restorative dentistry to visualize and simulate the final outcome of a dental treatment before the actual procedure is performed.

As already said, the obtained wax-up simulates all the individual's intraoral structures, part of the individual's intraoral structures or a single intraoral structure. Advantageously, the milled/printed intraoral structures correspond to the oral structures shown in the wax-up.

A milling and/or printing module 60, which will be described later, is advantageously used for the milling or printing step 18.

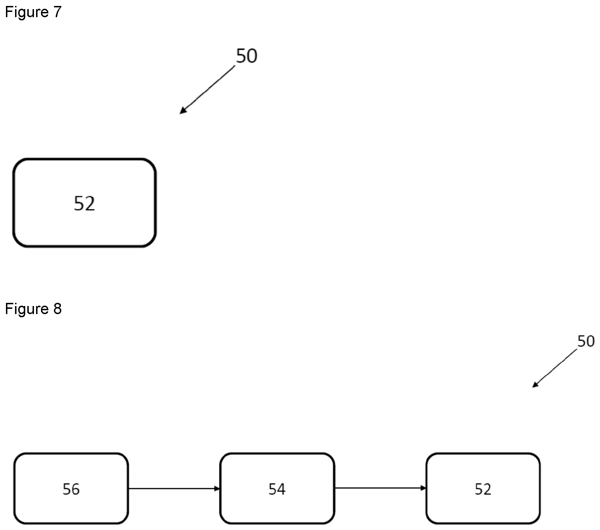

Now, an installation 50 for implementing the method 10 will be described with reference to figures 7 to 10.

As shown in figure 7, the installation 50 comprises a wax-up generation module 52 configured to digitally and automatically model the proposed position and/or shape of individual's intraoral structures. The wax-up generation module is in particular configured to take into account the determined performance of the jaw. In other words, the wax-up generation module 52 is configured to carry out step 12 of the method 10.

The wax-up generation module 52 is advantageously provided with a first artificial intelligence model which is able to propose a wax-up modelling the position and/or shape of the intraoral structures in order to give the individual's jaw the determined performance sought in step 12 of method 10.

In a non-limiting embodiment, the wax-up generation module 52 is based on a hybrid approach combining traditional programming algorithms and artificial intelligence.

The wax-up generation module 52 may use a mathematical approach. A central algorithm is designed to perform a succession of adjustments and generations according to established mathematical rules in order to build the wax-up based on the determined performance of the jaw function.

In addition, the wax-up generation module 52 may allow manual adjustments of the proposed wax-up. To that end, the wax-up generation module 52 may comprise a human-machine interface allowing the person using the wax-up generation module 52 to input these adjustments. Such adjustments may be added after having obtained the wax-up based on the determined performance of the jaw function (cf. step 12 of method 10). This allows the professional planning the dental treatment to manually modify the proposed wax-up based on the knowledge and/or preferences of the professional and/or the individual intended to receive the dental treatment. The adjustments can also be imposed by the professional to the wax-up generation module 52 prior to the obtention of the wax-up based on the determined performance, so that the obtained wax-up already integrates these adjustments from the moment it is proposed by the wax-up generation module 52. Examples of adjustments that may be input by the person using the wax-up generation module 52 are:
- value of the increase in the vertical dimension of the jaw;
- implementation of a 3D smile design;
- determination of the teeth to be kept and the teeth to be extracted;
- superimposition of a cone beam computed tomography (CBCT) scan in order to correlate the wax-up with the anatomical bone corridors.

The main algorithm of the wax-up generation module 52 can in addition be enhanced by decision blocks based on machine learning models. These algorithms are trained using data from wax-ups previously produced by professionals. This allows to optimize the most complex wax-up design processes. The algorithms may also be trained with bibliographic data. The data required for this training is pre-processed to extract features that can be used by the machine learning models, whether these are geometric measurements, quantifications based on X-rays, 3D STL captures, or jaw tracking data obtained by the jaw tracker module 56 that will be described later. The data required for this training can also come from libraries updated with data provided by dental health professionals, such data being obtained from real cases, for example.

The wax-up generation module 52 is able to propose a wax-up simulating the whole, some or only one individual's intraoral structure(s), depending on the extent of the planned dental treatment as explained above with reference to step 12 of the method 10.As also explained above in relation to the method 10, the wax-up generated by the wax-up generation module 52 is based on the actual performance of the jaw function of the individual when this is considered satisfactory. If the actual performance of the jaw function of the individual is not considered satisfactory, the wax-up proposed by the wax-up generation module 52 is based on a modified performance of the jaw function which is considered satisfactory according to the individual's jaw physiology or, alternatively, according to the bibliography. In addition, when the actual performance of the jaw function of the individual is not determined, the wax-up may be based on bibliographic data as explained before.

According to one embodiment, when the wax-up includes at least two teeth, the wax-up includes a prior segmentation, that independently separates each tooth, each tooth being generated as a separate body in the form of an independent STL file.

According to one embodiment, the wax-up generation module 52 further includes an output module adapted to generate and export the processed data in standardized formats usable by dental surgeons and dental laboratories.

For example, the standardized format is STL, the corrected wax model is automatically exported in this format and is adapted to reflect the final geometry with all occlusal corrections and preserve mesh resolution and segmentation.

The exported STL file of the wax-up is adapted to be reloaded into software such as Exocad and/or 3shape, and/or any other design software.

As an example, the STL file of the wax-up mesh contains several million polygons.

The wax generation module 52 and the jaw function module 54 are adapted to utilize advanced parallel computing techniques and available hardware capabilities.

In the embodiment shown in figure 8, the installation 50 further comprises a jaw function module 54 configured to determine an actual performance of the jaw function of the individual. This module 54 advises the person planning the dental treatment on maintaining or modifying the functional shield of the individual's mandible and/or the mandible position and/or the teeth position.

The jaw function module 54 is provided with a software able to determine the performance of the jaw function of the individual to be treated. This software may include another artificial intelligence model trained to determine parameters of the performance of the jaw function, to determine if this is a satisfactory performance, and, when it is not a satisfactory performance, to propose corrections of the parameters having an impact on the performance of the jaw function.

The jaw function module 54 also includes an occlusal correction function adapted to offer global vertical displacements which aim to correct occlusal defects by a rigid translation of a tooth strictly along the Z axis, illustrated in [Fig. 4].

According to one embodiment, the occlusal correction function is further configured to trigger a global vertical displacement only when the deviation or the functional interference associated with the considered tooth exceeds a threshold of 1.0 mm.

Automatic vertical corrections are restricted to cases in which the required translation remains within a predefined admissible range, for example between 1.0 mm and 1.5 mm.

Any correction requiring a displacement greater than a predetermined threshold, for example 1.5 mm, is automatically excluded from the correction process. If, after the automatic vertical translation, the corrected tooth still fails to establish a functional antagonist, no additional vertical adjustment is applied.

The jaw function module 54 also includes a material removal function adapted to apply Boolean subtraction or targeted reduction on areas of interpenetration. Volumes exceeding the 0.1 mm tolerance will be automatically identified and removed.

The material removal function includes an interpolation algorithm adapted to analyze local geometry to reconstruct a smooth, anatomically coherent transition surface.

The jaw function module 54 also includes a local positive offset function adapted to achieve a local positive offset which generates a controlled extrusion, constrained by the adjacent morphology and allowing to avoid an excessive volume.

The jaw function module 54 also includes a volumetric interpolation function adapted to reconstruct anatomical continuity and then perform iterative morphological smoothing corrections.

In one embodiment, after each correction iteration, a new dynamic simulation made by the jaw function module 54 is performed, the corrective process is repeated until a functional, harmonized wax-up is achieved.

In one example, the version selected for the wax-up is exported as an STL file with a digital report detailing, for each tooth, the type of correction (removal or addition), the depth and/or thickness of the modification, and the occlusal validation. In one embodiment, the jaw function module 54 further includes a machine equipped with a dedicated high-performance GPU suitable for performing the most computationally intensive tasks. In one example, these tasks include collision detection and Boolean subtraction.

The high-performance GPU is suitable for using compatible parallel computing libraries such as CUDA and/or OpenCL.

In one embodiment, the jaw tracker 56 module's algorithms are adapted to meet speed requirements compatible with clinical workflow, using optimized data structures such as BVH trees or KD trees.

The jaw function module 54 may be able to determine the mandible position and/or the mandible movement from the images recorded during step 16 of the method 10. In addition, the jaw function module 54 may be able to determine anterior guidance by several methods, like the Slavicek triangle.

The Slavicek triangle is a diagnostic tool that allows to analyze the functional relationships between the teeth, jaw, and temporomandibular joint (TMJ). This tool helps in evaluating how the upper and lower teeth come together, which allow determining the extent of overbite and overjet.

The jaw function module 54 may be also configured to determine the occlusal plane. To this end, the jaw function module 54 can be trained to calculate the occlusal plane using the Cooperman plane or the Monson sphere. The Monson sphere is positioned according to the anterior guidance and the position of the mandible.

The jaw function module 54 advantageously allows the user to input certain parameters that may not be determined from the analysis of the recorded images or that are desirable in the final result of the treatment. Examples of parameters that may be input are the need to increase the vertical dimension of occlusion, to remove or change the position/shape of certain teeth, etc.

Other parameters that may be input are certain notable anatomical elements, present on intraoral scans and/or CBCT.

The jaw function module 54 can also be trained to automatically position the maxilla and notable anatomical features on the functional and dynamic digital model of the entire jaw using either the Camper plane or the HIP plane, respectively. The HIP plane may require the presence of significant anatomical landmarks on intraoral CT and/or CBCT scans.

The installation 50 may also comprise a jaw tracker module 56. In some cases, the jaw tracker module 56 may be integrated in the jaw function module 54. In other cases, the jaw tracker module 56 and the jaw function module 54 are independent modules. The jaw tracker module 56 can be included in a smartphone application.

The jaw tracker module 56 is configured to analyse the movements of the individual's jaw. To that end, the jaw tracker module 56 may be provided with a software able to analyse images (pictures, videos, etc, for example .stl or ;ply files) recorded during step 16 of the mandible's movements, namely:
- the opening and/or closing trajectory of the individual's mandible; and/or
- the dynamic occlusion of the individual's mandible and maxilla, which, as explained before, may include right-left laterotrusion movements and forward-backward movements of the individual's mandible.

Since the jaw tracker module 56 may be included in a smartphone application, the images may be recorded with a smartphone and treated by the jaw tracker module 56 installed in the same smartphone.

The software of the jaw tracker module 56 may be able to recognize the teeth and link them to previously recorded digital impressions of the patient's maxilla and mandible (e.g..stl or .ply files). In some cases, the individual's own teeth and/or other intraoral structures may serve as markers for the software to recognize each tooth. In other cases, the individual may wear some markers on at least some teeth and/or other intraoral structures during image recording, these markers being recognizable by the software of the jaw tracker module 56, so that it can identify each tooth.

To analyse the mandible's movements, the jaw tracker module 56 may be configured to plot a diagram of the jaw's movements. Two examples of these kinds of diagram are shown in figures 9A and 9B. As shown in this figure, each diagram 57A, 57B comprises an arrow 58, 59. The vertical part 58-1, 59-1 of each arrow 58, 59 represents the opening and/or closing trajectory of the individual's mandible. The circumflex part 58-2, 59-2 of each arrow 58, 59 represents the right-left laterotrusion movements. In the case of diagram 57A, the vertical part 58-1 has no deviation, which means that the opening and/or closing trajectory of the individual's mandible is satisfactory. The circumflex part 58-2 is almost symmetrical to the right and left in relation to the vertical part 58-1, which means that the left and right laterotrusion movements are harmonious and therefore satisfactory. On the contrary, in the case of diagram 57B, the vertical part 59-1 presents a deviation, which means that the opening and/or closing trajectory of the individual's mandible may be improved. The circumflex part 59-2 is totally asymmetrical to the right and left in relation to the vertical part 59-1, which means that the left and right laterotrusion movements are not harmonious and therefore, they are not satisfactory.

The jaw tracker module 56 may be also configured to analyze the static occlusion of the individual's mandible and maxilla.

All these actions of the jaw tracking module 56 make it possible to obtain a 3D model of the dynamics of the individual's jaw. In particular, animated 3D models of the individual's jaw can be obtained and displayed on a graphical interface.

The jaw tracker module 56 may be provided with another artificial intelligence model in order to accomplish all the above-mentioned actions. This artificial intelligence model may be in particular be trained to:
- analyze images recorded during step 16 of the method 10, and/or
- recognize each tooth of the individual to be treated (when the individual is wearing markers and when the teeth or other intra-oral structures are themselves the markers); and/or
- superimpose each individual tooth to previously recorded digital impressions of the patient's maxilla and mandible; and/or
- plot the diagram of the jaw's movements.

As an example, the artificial intelligence model included in the jaw tracker module 56 is based on machine learning and/or computer vision.

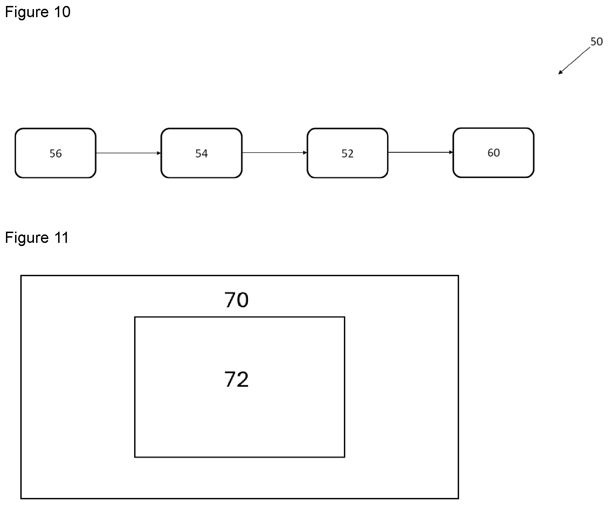

As shown in the non-limiting example of figure 10, the installation 50 further comprises a milling and/or printing module 60. This module 60 is configured to mill and/or print a dental prothesis, and/or a dental orthosis, and/or orthodontic devices, and/or guides, and/or mock-ups considering the position and/or shape of the intraoral structures in the wax-up.

For treatment plans involving dental implants, the dental implants are planned and surgically placed according to the functional wax-up generated by the wax-up generation module 52. At the end of the implant surgery, to rehabilitate the implants quickly with a fixed temporary solution, a digital impression of the situation with dental implants can be recorded using an intraoral scanner. The file obtained (which may be, but is not limited to, a .stl or .ply file) can then be used to superimpose the new situation including the dental implants with the wax-up generated by the wax-up generation module 52. This allows a digital file (for example, a .stl file) of a temporary crown or bridge to be prepared, adapted to the dental implants fitted and to the wax-up obtained during step 12 of method 10.

In prior art, the production time for a .stl file for a full bridge in an arch is advantageously 30 minutes, the production time for a .stl file for a tooth or a small bridge is 15 minutes. With the method 10 and the installation 50, these .stl files are obtained instantly. The .stl file can then be used and milled or printed by the milling and/or printing module 60.

All these modules 52, 54, 56, 60 and the operations they are capable of performing can be deployed on a cloud after having imported the initial data, which may include digital files (usually a .stl), images, a 3D smile design, a CBCT scan, etc.

By "deployed on a cloud", we mean that the modules 52, 54 and 56 do not require any mechanical device, and are adapted to perform their functions in software. For the module 60, all the information it needs to mill or print is software-based.As is clear from the present disclosure, the method and the installation described make it possible to obtain a wax-up that takes into account the jaw function of the individual to be treated. The wax-up is therefore not only based on aesthetic aspects of the smile, but also on the other parameters that have an impact on the satisfactory functioning of the individual's jaw and so, of the manducatory apparatus. The chances of success and durability of the planed dental treatment are therefore increased. **In** addition, these aesthetical, functional, and long-term results are obtained by a method which is simple and efficient in its application.

Figure 11 represents a computer program 70. The computer program 70 comprises instructions 72 which, when the program is executed by a computer, cause the computer to carry out the method 10. The computer program 70 may comprise an artificial intelligence model, for example a machine learning model or a machine learning data set. The computer may be the installation 50 or part of the installation 50 described above.

The computer program 70 may be configured to launch the wax-up generation module 52, and/or the jaw function module 54, and/or the jaw tracker module 56. The computer program 70 may also be configured to launch the milling and/or printing module 60.

The wax-up generation module 52, and/or the jaw function module 54, and/or the jaw tracker module 56 may in some cases being included in the program 70, but in other cases they are included in the installation 50 as described before.

While exemplary embodiments of the disclosed method, installation and computer program have been described above, it will be understood by those skilled in the art that various changes, omissions and/or additions may be made, and equivalents may be substituted for elements thereof. In addition, many modifications may be made to adapt a particular situation or material to the teachings of this disclosure without departing from the scope thereof. Therefore, it is intended that the method, installation and computer program described above are not limited to the particular embodiments disclosed above, but that the they will include all embodiments falling within the scope of the appended claims. Moreover, unless specifically stated any use of the terms first, second, etc. do not denote any order or importance, but rather the terms first, second, etc. are used to distinguish one element from another.

## Claims

1. Method (10) for planning a dental treatment on an individual, the method (10) comprising digitally and automatically obtaining (12) a wax-up built based on a determined performance of the jaw function of the individual, the wax-up comprising a proposed position and/or shape of the individual's intraoral structures.

2. Method (10) according to claim 1, further comprising determining (14) an actual performance of the jaw function of the individual, the determined performance on which the wax-up is based corresponding to said actual performance or to a modified performance of the jaw function of the individual with respect to said actual performance.

3. Method (10) according to claim 2, wherein determining (14) the actual performance of the jaw function includes obtaining a diagram (50) of the jaw's movement.

4. Method (10) according to any one of claims 2 or 3, wherein determining (14) the actual performance of the jaw function includes determining (14-1) a mandible position and/or a mandible movement of the individual.

5. Method (10) according to claim 4, wherein the mandible position is determined by analysing:
- an opening and/or closing trajectory of the individual's mandible; and/or
- a dynamic occlusion of the individual's mandible and maxilla; and/or
- a static occlusion of the individual's mandible and maxilla.

6. Method (10) according to any one of claims 2 to 5, wherein determining (14) the actual performance of the jaw function of the individual comprises determining (14-2) anterior guidance.

7. Method (10) according claim 6, wherein determining (14-2) the anterior guidance comprises obtaining an overjet (20) value and/or an overbite (22) value of the individual's jaw.

8. Method (10) according to any one of claims 2 to 7, wherein determining (14) the actual performance of the jaw function of the individual comprises determining an occlusal plane (14-3).

9. Method (10) according to any one of the previous claims, further comprising one or more of the following features:
- the wax-up includes a corrected functional shield of the individual's mandible and/or a corrected mandible position and/or a corrected teeth position when the actual performance of the jaw function is unsatisfactory;
- at least an artificial intelligence model is employed for digitally and automatically obtaining the wax-up.

10. Method (10) according to claim 2 and any one of claims 3 to 9, wherein at least an artificial intelligence model is employed for determining the actual performance of the jaw function.

11. Method (10) according to any one of the preceding claims, further comprising one or more of the following features:
- the wax-up simulates all individual's intraoral structures or a part of the individual's intraoral structures;
- the method (10) further comprises milling or printing (18) a dental prothesis, and/or a dental orthosis, and/or orthodontic devices, and/or guides, and/or mock-ups considering the position and/or shape of the intraoral structures in the wax-up.

12. Method (10) according to any one of the preceding claims, further comprising registering (16) an image of at least part of the individual's jaw, preferably at least part of the jaw in movement.

13. Installation (50) for implementing the method (10) according to any one of the previous claims, the installation comprising a wax-up generation module (52) configured to digitally and automatically model the proposed position and/or shape of individual's intraoral structures, the wax-up generation module (52) being configured to take into account said determined performance of the jaw function when modelling said wax-up.

14. Installation (50) according to claim 13, further comprising one or more of the following features:
- a jaw function module (54) configured to determine an actual performance of the jaw function of the individual;
- a jaw tracker module (56) configured to obtain a 3D model of individual's jaw dynamics from analysing an image of at least part of the jaw of the individual;
- a milling and/or printing module (60) configured to mill and/or print a dental prothesis, and/or a dental orthosis, and/or orthodontic devices, and/or guides, and/or mock-ups considering the position and/or shape of the intraoral structures in the wax-up.

15. Computer program (70) comprising instructions (72) which, when the program is executed by a computer, cause the computer to carry out the method (10) of any one of claims 1 to 12.
